# EUROPEAN PATENT SPECIFICATION

(11) **EP 4 115 943 B1**
(45) Date of publication and mention of the grant of the patent: **22.11.2023**
(21) Application number: 21183758.8
(22) Date of filing: 05.07.2021
(51) Int. Cl.: A61N 1/36, A61N 1/372, H04R 25/00, A61B 5/12

(54) **HEARING PROSTHESIS SYSTEM**
HÖRPROTHESENSYSTEM
SYSTÈME DE PROTHÈSE AUDITIVE

(43) Date of publication of application: 11.01.2023
(73) Proprietor: Advanced Bionics AG, 8712 Staefa (CH)
(72) Inventor: Thiemann, Joachim, 30179 Hannover (DE); Koning, Raphael, 30900 Wedemark (DE); Koka, Kanthaiah, Valencia, CA 91355 (US)
(74) Representative: Schwan Schorer & Partner mbB

(56) References cited:
- US-A1- 2016 175 591
- US-A1- 2016 235 986
- US-A1- 2018 015 287
- US-A1- 2020 054 877

## Description

The invention relates to a hearing prosthesis system comprising an implantable electrode array, a cochlear implant coupled to the electrode array, and a processing unit communicatively coupled to the cochlear implant.

Hearing prosthesis systems may use electrical stimulation of the cochlea via the electrode array only or, for patients with residual hearing, the system in addition may apply acoustic stimulation (such bimodal stimulation systems are known as EAS-systems). Hearing prosthesis systems have to be adapted to the individual patient by fitting sessions, wherein fitting parameters, such as electrical stimulation or acoustic stimulation threshold levels, acoustic amplification gains for EAS-patients, etc., are determined and set. Since the condition of the patient's hearing and electrode conditions may vary over time, regular assessment of the performance of the hearing prosthesis system by monitoring the operating parameters is desirable. In particular, shifts of the steady state parameters should be determined and the normal viability thereof should be tracked.

Assessment of the system performance may include objective measurements, such as measurement of electrocochleography (ECochG) thresholds, neural response imaging (NRI) thresholds and cortical responses, via the electrode array, wherein the measured electrical signals are transmitted via back-telemetry to the external processing unit. Such measurements involve electrical stimulation via the electrode array and/or acoustic stimulation (in case of EAS systems). Such hearing stimulation often involves an audible or at least noticeable percept that is not consistent with normal stimulation pattern and therefore is considered unnatural by user. As a consequence, such system performance measurements typically are conducted only at visits of the patient to the hearing care professional (HCP), which may be infrequent and/or irregular.

WO 2020/044307 A1 relates to a hearing prosthesis system wherein stimulation for objective measurements of system performance is applied during times when a state of sleep of the patient has been determined, so as to avoid that the patient is disturbed by the hearing stimulation required for the objective measurements.

US 2017/0304632 A1 relates to a cochlear implant system wherein automated ECochG testing outside of a clinical setting is performed using the normal sounds that a patient hears in the course of the daily life. To this end, ambient sound signals received by the cochlear implant system during normal operation are analyzed to identify portions of sound signals that are suitable for performing an ECochG measurement using the implanted electrode array. The ECochG measurements are used for assessing the patient's residual hearing, so as to initiate corrective actions if a change of the residual hearing is detected.

Other hearing prosthesis systems are known from US2018/015287 A1, US2016/235986 A1, US2020/054877 A1 and US2016/175591 A1.

It is an object of the invention to provide for a hearing prosthesis system which allows for relatively regular monitoring of the system performance in a patient-friendly manner. According to the invention, this object is achieved by a hearing prosthesis system as defined in claim 1.

The invention proposes to utilize a user interaction audio signal which is indicative of an interaction of the patient with the hearing prosthesis system as a test audio signal for applying perceivable hearing stimulation to the patient according to the user interaction signal and to detect, via the electrode array, a neural response of the patient to such hearing stimulation. Thereby objective system performance measurements can be conducted in an unobtrusive manner on a relatively frequent basis during normal use, in particular outside a clinical setting, of the hearing prosthesis system.

In some implementations,, the user interaction audio signal is a feedback message signal indicative of a user interaction on a user interface of the hearing prosthesis system or a status message signal indicative of a change in a condition of the hearing prosthesis system. For example, the feedback message signal may be indicative of a user action resulting in a hearing program change, a volume increase, a volume reduction or locking or unlocking of the implant. For example, the status message signal may be indicative of a wireless connection or disconnection of the external processing unit with external device, or it may be indicative of a low battery status.

In some implementations, the user interaction audio signal is selected such that is perceivable by the patient as a standard sound associated with the respective feedback message or a status message.

In some implementations, the feedback message signal is in response to a user action on an operator control. For example, the operator control may be a mechanical element disposed on an external housing including the processing unit; for example, the operator control may be a button or a key to be pressed. According to another example, the operator control may be provided on a remote control device communicatively coupled with the processing unit; for example, the operator control may be implemented as a touch screen of the remote control device. According to still another example, the feedback message signal may be in response to a user action on an operator control of an accessory device communicatively coupled to the processing unit; for example, the accessory device may be smartphone and the user action may be locking or unlocking of a touchscreen of the smartphone.

In some implementations, the processing unit is configured to apply the perceivable hearing stimulation corresponding to the user interaction audio signal at a level within the comfort range.

In some implementations, the user interaction audio signal includes sinusoidal tones and/or frequency sweeps.

In some implementations, the recording of the neural response to the user interaction audio signal includes neural response imaging (NRI) threshold measurements, electrocochleography (ECochG) threshold measurements, cortical response measurements and/or electrode impedance measurements.

In some implementations, the processing unit is configured to apply said perceivable hearing stimulation to the patient according to the user interaction audio signal as electrical stimulation via the electrode array. For example, the processing unit may be configured to select only some of the electrodes of the electrode array which are found to characterize the fitting curves and to use only the selected electrodes for the electrical stimulation according to the user interaction audio signal.

In some implementations, the hearing prosthesis system is an EAS system including an electroacoustic output transducer, wherein the processing unit is configured to apply said perceivable hearing stimulation to the patient according to the user interaction audio signal as acoustic stimulation via the electroacoustic output transducer. Fore example, the recording of the neural response to the user interaction audio signal may include measurement of ECochG signals.

In some implementations, the processing unit is configured to automatically adjust fitting parameters of the hearing prosthesis system according to the recorded neural response.. For example, the processing unit may be configured to automatically adjust stimulation threshold levels of the hearing prosthesis system according to the recorded neural response, and wherein the user interaction audio signal may result in hearing stimulation suitable for determining the respective threshold from the recorded neural response; for example, the recording of the neural response to the user interaction audio signal may include cortical response measurements for adjustment of electrical stimulation thresholds.

In some implementations, the processing unit is configured to store data corresponding to neural responses recorded over a time period for later use in manual adjustment of fitting parameters.

In some implementations, the processing unit is integrated an external unit. For example, the external unit may be configured to be worn at the patient's head; for example, the external unit is a BTE unit or a headpiece.

In some implementations, the cochlear implant and the processing unit are coupled via an inductive transcutaneous link, wherein the neural response to the user interaction audio signal is supplied to the processing unit by back telemetry via the inductive transcutaneous link.

Preferred embodiments of the invention are defined in the dependent claims.

Hereinafter, examples of the invention will be illustrated by reference to the attached drawings, wherein:
- Fig. 1: is a schematic illustration of a hearing prosthesis system according to the invention during a neural response measurement;
- Fig. 2: is a schematic illustration of an example of a hearing prosthesis system according to the invention
- Fig. 3: is a schematic illustration of an electrode array of the system of Figs. 1 and 2, as inserted in a cochlea, during a neural response measurement; and
- Fig. 4: is a schematic illustration of an example of user interactions resulting in a user interaction audio signal to be used in a neural response measurement.

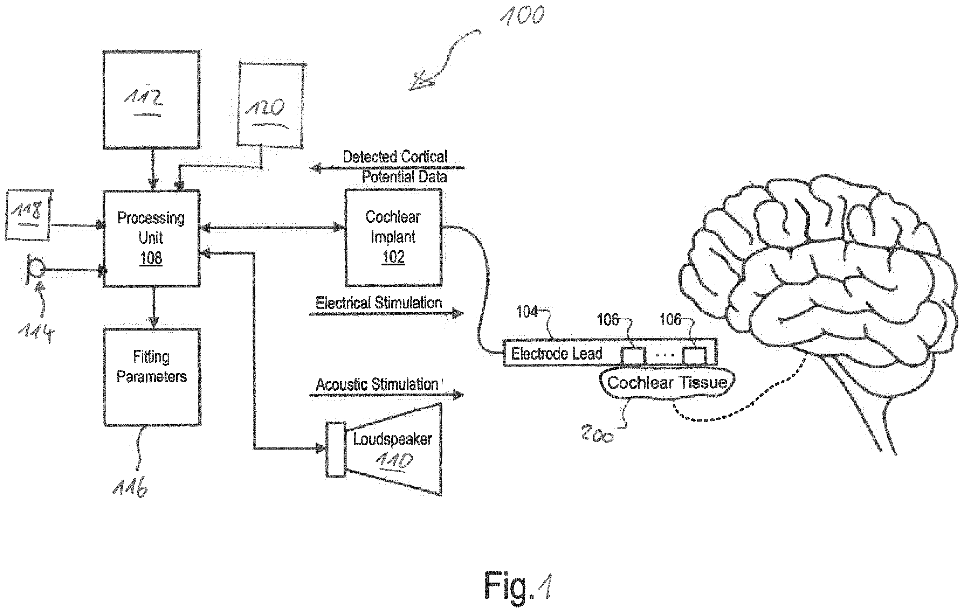

Fig. 1 is a schematic illustration of functional components of an example of a cochlear implant system 100, comprising a cochlear implant 102 to be implanted within a patient, an electrode array (or electrode lead) 104 comprising a plurality of electrodes 106 to be implanted within a cochlea 200 of the patient and coupled to the cochlea implant 102, and a processing unit 108 communicatively coupled to the cochlea implant 102. The system may further comprise an electroacoustic output transducer 110, such as a loudspeaker, coupled to the processing unit 108 for providing acoustic stimulation to the patient's hearing, a user interface 112 coupled to the processing unit 108, a microphone 114 coupled to the processing unit 108 for capturing input audio signals from ambient sound, and a memory 116 coupled to the processing unit 108 for storing fitting parameters and other system parameters.

The processing unit 108 is configured to direct the cochlear implant 102 to apply stimulation to the cochlea 200 via the electrodes 106 of the electrode array 104 and to direct the electroacoustic output transducer 110 to apply acoustic stimulation to the patient so as to stimulate the residual hearing of the patient. To this end, the processing unit 108 generates corresponding electrical stimulation signals which are supplied to the cochlear implant 102 and acoustic stimulation signals which are supplied to the electroacoustic output transducer 110. The processing unit 108 may generate such stimulation signals based on audio input signals received from the microphone 114 or an audio input 118 which may be, for example, a communication interface to an accessory device, such as a smartphone or an audio streaming device, so as to stimulate, in a normal operation mode, the hearing of the patient according to the input audio signals to make the input audio signals perceivable by the patient.

Further, the processing unit 108 may generate electrical and/or acoustic stimulation signals in response to a user interaction with the user interface 112, so as to provide a feedback message, such as a characteristic tone, to the patient, and/or the processing unit 108 may generate the stimulation signals according to a status change of the system, such as a wireless connection or disconnection of the processing unit with an external device, such as a smartphone, or to indicate a low battery status. To this end, the processing unit 108 may be coupled to respective sensors 120 which sense certain system conditions such as battery status.

Electrical stimulation via the electrodes 106 and/or acoustic stimulation or via the output transducer 110 results in a neural response of the patient to such hearing stimulation, which neural response can be detected via the electrode 106 of the electrode array, with the detected neural response signals being supplied via the cochlea implant 102 to the processing unit 108 for analysis of neural response signals.

As already mentioned above, the processing unit 108 may generate a user interaction audio signal which is indicative of an interaction of the patient with the hearing prosthesis system 100, and apply perceivable hearing stimulation to the patient according to the user interaction audio signal. The user interaction audio signal, for example, may be a feedback message signal indicative of a user action, in particular an action by the patient, on the user interface 112 or a status message signal indicative of a change in a condition of the hearing prosthesis system, such as sensed by the sensors 120.

The processing unit 108 further may record, via the electrode array 104 and the cochlear implant 102, the neural response to such hearing stimulation according to such user interaction audio signal, thereby utilizing the user interaction audio signal as a test audio signal. In this way hearing prosthesis performance can be monitored by performing relatively frequently active objective neural response measurements in a relatively unobtrusive way, i.e. without disturbing the patient by perception of unexpected or unusual hearing sensations, so that implant health and operation parameter fitting can be monitored during normal use of the hearing prosthesis by the patient. In particular, the test audio signals thereby can be designed in a manner that the patient experiences the expected perception of user interface feedback and/or status information. In other words, the patient perceives usually expected standard sounds/signals only. In particular, any signal tone may consist of suitable measurement stimuli for purposes such as fitting of the electrical and/or acoustical path. In other words, a user interaction with the hearing prosthesis actually triggers an active measurement with a stimulus at a level to be clearly perceivable, while the patient actually does not recognize that a neural response measurement presently takes place.

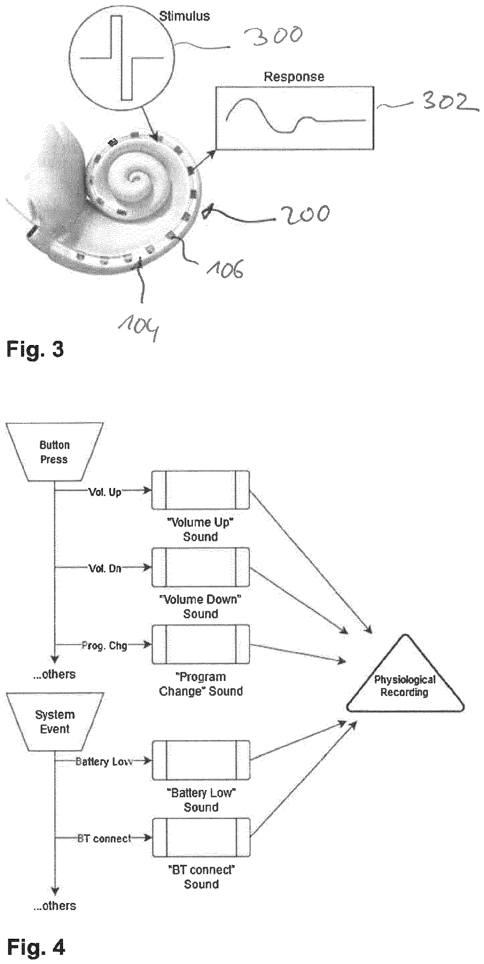

Fig. 3 is an illustration of a neural response measurement, wherein the electrode array 104 with electrodes 106 is shown as implanted within a cochlea 200. An example of a stimulation signal applied to an electrode 106 is schematically shown at 300 and a corresponding neural response signal collected by another one of the electrodes 106 in response to the stimulation signal 300 as indicated at 302.

The perceivable hearing stimulation corresponding to the user interaction audio signal may be applied at a level within the comfort range of the patient.

In some examples, the user interaction audio signals may include sinusoidal tones and/or frequency sweeps.

In some implementations, known previously used measurement signals may be modified so as to be clearly audible to the patient and create a pleasant perception which is similar or equal to a standard sound perception typical for user interaction with the hearing prosthesis system. Further, known previously used common user interaction audio signals, such as user interface feedback sounds, may be modified so as to be suitable for performing neural response measurements, while still being perceivable as a standard sound. Generally, also new stimuli may be designed, as long as the patient feels to perceive some kind of standard user interaction sound, while the patient should not have the feeling of a measurement being performed.

Examples of interactions of the patient with the hearing prosthesis system are schematically shown in Fig. 4, according to which user interaction with the user interface 112 may include "volume up", "volume down" or "program change" commands by the patient, resulting in the processing unit 108 generating corresponding user feedback sounds. Another example is an implant lock or unlock command by the patient. For example, the "implant lock" sound may be a tone sequence resulting from stimulation on selected ones of the electrodes 106. A "volume up" sound may include ascending measurements on even-numbered electrodes, and a "volume down" sound may comprise descending measurements on odd-numbered electrodes. According to a further example, a "program change" sound may be a long tone resulting from stimulation on a selected electrode depending on the selected program. In all cases, the current level may depend on the presently selected volume, which, in case of "volume up" or "volume down" commands may be the present volume level or the new volume level. Examples of system events resulting in a status message signal may be "battery low", resulting in a characteristic "battery low" sound, or "Bluetooth connect" resulting in a corresponding characteristic "Bluetooth connect" sound.

According to another example, the interaction of the patient with the user interface 112 may be the locking or unlocking of a screen of a smartphone communicatively coupled, for example, via a Bluetooth connection, to the processing unit. In this case, the processing unit 108 may perform, for example, an electrode impedance measurement when such unlocking or locking of the smartphone screen happens, which will cause a noticeable short sound which equals or is similar to a sound typically used for indicating screen locking / unlocking. The processing unit 108 then may verify that the resulting impedance measurements are within the expected range or, alternatively or in addition, it may store the measurement values in the memory 116 for long-term trend analysis.

In some implementations, the feedback message signal may be in response to a user interaction on an operator control, which, for example, may be a mechanical element disposed on an external housing including the processing unit 108; in particular, the operator control may be a button or a key to be pressed. In another example, the operator control may be provided on a remote control device communicatively coupled with the processing unit 108; for example, the operator control in this case may be implemented as a touchscreen of the remote control device. In some implementations, the feedback message signal may be in response to a user action on an operator control of an accessory device coupled to the processing unit 108; in particular, the accessory device may be a smartphone and the user action may be locking or unlocking of a touchscreen of the smartphone, as already mentioned above.

The recording of the neural response to the user interaction audio signal may include NRI threshold measurements, ECochG threshold measurement, cortical response measurements and/or electrode impedance measurements.

For applying electrical stimulation according to the user interaction audio signal the processing unit 108 may select some of the electrodes 106 of the electrode array 104, namely those electrodes which are found to characterize the fitting curves of the hearing prosthesis system. Accordingly, it is sufficient to use only these selected electrodes for the electrical stimulation according to the user interaction audio signal, i.e., for the neural response measurements; there is no need to use all electrodes.

In case that the hearing prosthesis system provides also for acoustic stimulation, measurement of ECochG signals is particularly useful.

For example, an acoustic sound could be presented via the output transducer 110 at different frequencies and the resulting ECochG signals may be measured via the electrodes 106. With the measured values the acoustic threshold could be estimated and compared to the present setting, and subsequently could be adjusted automatically if required.

Electrical stimulation via the electrodes 106 may be used for evoking and measure cortical potentials. Such cortical potential measurements can be used for validating the electrical threshold levels and to automatically adjust the levels if required, as described, for example, in U.S. patent application 62/926,351 of the applicant of the present application.

In general, the recorded neural responses may be used by the processing unit 108 to automatically adjust all kinds of fitting parameters, not only threshold levels.

Alternatively or in addition, the processing unit 108 may store data corresponding to the neural responses recorded over a time period in the memory 116 for later use in manual adjustment of fitting parameters, for example, by a hearing care professional.

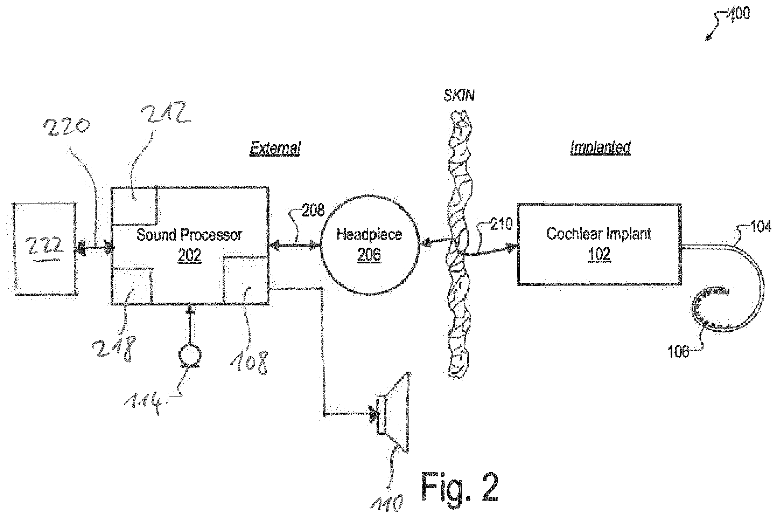

An example of a practical implementation of the system illustrated in Fig. 1 is schematically shown in Fig. 2, wherein the processing unit 108 forms part of an external unit, which in the example of Fig. 2 is formed by a sound processor 202 which also may include a wireless interface 218 for establishing a wireless connection 220 to an external device 222, such as a smartphone. The sound processor 202 further may include an integrated user interface 212 formed by an operator control, such as a button or key. The external device 222 may provide for an alternative or additional user interface. The sound processor 202 may be connected to an electroacoustic output transducer 110 which may be implemented as a device to be worn in the ear canal. The sound processor 202 may be connected via a connection 208 to a headpiece 206 which comprises a coil for establishing a wireless transcutaneous link 210 with a corresponding coil of the cochlear implant 102. The sound processor 202 may be designed to be worn at the patient's head, for example as a BTE unit; alternatively, it may be integrated within the headpiece. According to another example, the sound processor 202 may be implemented as a body worn unit which is not worn at the head.

The neural response signals detected by the electrodes 106 may be supplied to the sound processor 202 by back-telemetry via the inductive transcutaneous link 210.

## Claims

1. A hearing prosthesis system comprising
an electrode array (106) configured to be implanted within a patient;
a cochlear implant (102) coupled to the electrode array and configured to be implanted within the patient; and
a processing unit (108) communicatively coupled to the cochlear implant;
wherein the processing unit is configured to:
direct the cochlear implant to apply stimulation to a cochlea of the patient via the electrode array; and
detect, via the electrode array, a neural response of the patient to hearing stimulation;
and **characterised in that**:
the processing unit is further configured to:
generate a user interaction audio signal indicative of an interaction of the patient with the hearing prosthesis system (100) and apply perceivable hearing stimulation to the patient according to the user interaction audio signal, and
record, via the electrode array and the cochlear implant, the neural response to said hearing stimulation according to the user interaction audio signal, thereby utilizing the user interaction audio signal as a test audio signal.

2. The system of claim 1, wherein the user interaction audio signal is a feedback message signal indicative of a user action on a user interface (112, 212, 222) of the hearing prosthesis system or a status message signal indicative of a change in a condition of the hearing prosthesis system (100).

3. The system of one of the preceding claims, wherein the user interaction audio signal is selected such that is perceivable by the patient as a standard sound associated with the respective feedback message or a status message.

4. The system of one of claims 2 and 3, wherein the status message signal is indicative of a wireless connection or disconnection of the processing unit (108) with an external device (222) or of a low battery status.

5. The system of one of claims 2 and 4, wherein the feedback message signal is indicative of a user action resulting in a hearing program change, a volume increase, volume reduction or an implant locking/unlocking.

6. The system of one of claims 2 to 5, wherein the feedback message signal is in response to a user action on an operator control (212, 222).

7. The system of one of the preceding claims, wherein the processing unit (108) is configured to apply the perceivable hearing stimulation corresponding to the user interaction audio signal at a level within the comfort range.

8. The system of one of the preceding claims, wherein the user interaction audio signal includes sinusoidal tones and/or frequency sweeps.

9. The system of one of the preceding claims, wherein the recording of the neural response to the user interaction audio signal includes neural response imaging (NRI) threshold measurements, electrocochleography (ECochG) threshold measurements, cortical response measurements and/or electrode impedance measurements.

10. The system of one of the preceding claims, wherein the processing unit (108) is configured to apply said perceivable hearing stimulation to the patient according to the user interaction audio signal as electrical stimulation via the electrode array (106).

11. The system of one of the preceding claims, wherein the hearing prosthesis system (100) is an Electro-Acoustic Stimulation, EAS, system including an electroacoustic output transducer (110), and wherein the processing unit is configured to apply said perceivable hearing stimulation to the patient according to the user interaction audio signal as acoustic stimulation via the electroacoustic output transducer.

12. The system of claim 11, wherein the recording of the neural response to the user interaction audio signal includes measurement of ECochG signals.

13. The system of one of the preceding claims, wherein the processing unit (108) is configured to automatically adjust fitting parameters of the hearing prosthesis system (100) according to the recorded neural response.

14. The system of claim 13, wherein the processing unit (108) is configured to automatically adjust stimulation threshold levels of the hearing prosthesis system (100) according to the recorded neural response, and wherein the user interaction audio signal results in hearing stimulation suitable for determining the respective threshold from the recorded neural response.

15. The system of claim 14, wherein the recording of the neural response to the user interaction audio signal includes cortical response measurements for adjustment of electrical stimulation thresholds.

## Patentansprüche

1. Hörprothesensystem mit
einer Elektrodenanordnung (106) zum Implantieren innerhalb eines Patienten;
einem Cochlea-Implantat (102), welches mit der Elektrodenanordnung gekoppelt und zum Implantieren innerhalb des Patienten ausgebildet ist; und
einer Verarbeitungseinheit (108), welche kommunikativ mit dem Cochlea-Implantat gekoppelt ist und ausgebildet ist, um:
das Cochlea-Implantat zum Anwenden von Stimulation auf eine Cochlea des Patienten über die Elektrodenanordnung zu veranlassen; und
über die Elektrodenanordnung eine neurale Reaktion des Patienten auf die Gehörstimulation zu erfassen;
und **gekennzeichnet dadurch, dass** die Verarbeitungseinheit ferner ausgebildet ist, um:
ein Nutzerinteraktions-Audiosignal zu erzeugen, welches bezeichnend für eine Interaktion des Patienten mit dem Hörprothesensystem (100) ist, und
wahrnehmbare Gehörstimulation auf den Patienten gemäß dem Nutzerinteraktions-Audiosignal anzuwenden und über die Elektrodenanordnung und das Cochlea-Implantat die neurale Antwort auf die Gehörstimulation gemäß dem Nutzerinteraktions-Audiosignal aufzuzeichnen, wodurch das Nutzerinteraktions-Audiosignal als ein Test-Audiosignal genutzt wird.

2. System gemäß Anspruch 1, wobei es sich bei dem Nutzerinteraktions-Audiosignal um ein Feedback-Nachrichtensignal, welches bezeichnend für eine Nutzeraktion betreffend eine Nutzerschnittstelle (112, 212, 222) des Hörprothesensystems ist, oder um ein Status-Nachrichtensignal handelt, welches bezeichnend für eine Veränderung eines Zustands des Hörprothesensystems (100) ist.

3. System gemäß einem der vorhergehenden Ansprüche, wobei das Nutzerinteraktions-Audiosignal so ausgewählt ist, dass es von dem Patienten als Standardklang, der mit der jeweiligen Feedback-Nachricht assoziiert ist, oder als Statusnachricht wahrgenommen werden kann.

4. System gemäß einem der Ansprüche 2 oder 3, wobei das Status-Nachrichtensignal bezeichnend für eine drahtlose Verbindung oder einen Verbindungsabbruch der Verarbeitungseinheit (108) mit einem externen Gerät (222) oder für einen schwachen Batteriestatus ist.

5. System gemäß einem der Ansprüche 2 oder 4, wobei das Feedback-Nachrichtensignal bezeichnend für eine Nutzeraktion ist, die zu einem Hörprogrammwechsel, einer Lautstärkeerhöhung, einer Lautstärkeverringerung oder einer Verriegelung oder Entriegelung des Implantats führt.

6. System gemäß einem der Ansprüche 2 bis 5, wobei das Feedback-Nachrichtensignal eine Reaktion auf eine Nutzerreaktion auf eine Operatorsteuerung (212, 222) darstellt.

7. System gemäß einem der vorhergehenden Ansprüche, wobei die Verarbeitungseinheit (108) ausgebildet ist, um die wahrnehmbare Gehörstimulation entsprechend dem Nutzerinteraktions-Audiosignal bei einem Pegel innerhalb des Komfortbereichs anzuwenden.

8. System gemäß einem der vorhergehenden Ansprüche, wobei das Nutzerinteraktions-Audiosignal sinusförmige Töne und/oder Frequenz-Sweeps beinhaltet.

9. System gemäß einem der vorhergehenden Ansprüche, wobei das Aufzeichnen der neuralen Reaktion auf das Nutzerinteraktions-Audiosignal Neural-Antwort-Bildgebung (NRI), Schwellwertmessungen, Elektrocochleographie (ECochG)-Schwellwertmessungen, Kortikal-Reaktionsmessungen und/oder Elektrodenimpedanzmessungen beinhaltet.

10. System gemäß einem der vorhergehenden Ansprüche, wobei die Verarbeitungseinheit (102) ausgebildet ist, um die wahrnehmbare Gehörstimulation auf den Patienten gemäß dem Nutzerinteraktions-Audiosignal als elektrische Stimulation über die Elektrodenanordnung (106) anzuwenden.

11. System gemäß einem der vorhergehenden Ansprüche, wobei es sich bei dem Hörprothesensystem (100) um ein elektroakustisches Stimulations(EAS)-System mit einem elektroakustischen Ausgangswandler (110) handelt, und wobei die Verarbeitungseinheit ausgebildet ist, um die wahrnehmbare Gehörstimulation auf den Patienten gemäß dem Nutzerinteraktions-Audiosignal als akustische Stimulation über den elektroakustischen Ausgangswandler anzuwenden.

12. System gemäß Anspruch 11, wobei das Aufzeichnen der neuralen Reaktion auf das Nutzerinteraktions-Audiosignal das Messen von ECochG-Signalen beinhaltet.

13. System gemäß einem der vorhergehenden Ansprüche, wobei die Verarbeitungseinheit (108) ausgebildet ist, um Anpassparameter des Hörprothesensystems (100) gemäß der aufgezeichneten neuralen Reaktion automatisch einzustellen.

14. System gemäß Anspruch 13, wobei die Verarbeitungseinheit (108) ausgebildet ist, um Stimulationsschwellwertpegel des Hörprothesensystems (100) automatisch gemäß der aufgezeichneten neuralen Reaktion einzustellen, und wobei das Nutzerinteraktions-Audiosignal zu einer Gehörstimulation führt, die geeignet ist, um den jeweiligen Schwellwert aus der aufgezeichneten neuralen Reaktion zu bestimmen.

15. System gemäß Anspruch 14, wobei das Aufzeichnen der neuralen Reaktion auf das Nutzerinteraktions-Audiosignal Kortikal-Reaktions-Messungen zum Einstellen von Schwellwerten für elektrische Stimulation beinhaltet.

## Revendications

1. Système de prothèse auditive comprenant :
un réseau d'électrodes (106) configuré pour être implanté chez un patient ;
un implant cochléaire (102) couplé au réseau d'électrodes et configuré pour être implanté chez le patient ; et
une unité de traitement (108) couplée de manière communicative à l'implant cochléaire ;
l'unité de traitement étant configurée pour :
diriger l'implant cochléaire pour appliquer une stimulation à une cochlée du patient par l'intermédiaire du réseau d'électrodes ; et
détecter, par l'intermédiaire du réseau d'électrodes, une réponse neuronale du patient à la stimulation auditive ; et **caractérisé en ce que** l'unité de traitement est en outre configurée pour :
générer un signal audio d'interaction d'utilisateur indiquant une interaction du patient avec le système de prothèse auditive (100) et appliquer une stimulation auditive perceptible au patient en fonction du signal audio d'interaction d'utilisateur, et enregistrer, par l'intermédiaire du réseau d'électrodes et de l'implant cochléaire, la réponse neuronale à ladite stimulation auditive en fonction du signal audio d'interaction d'utilisateur, utilisant ainsi le signal audio d'interaction d'utilisateur en tant que signal audio d'essai.

2. Système selon la revendication 1, le signal audio d'interaction d'utilisateur étant un signal de message de rétroaction indiquant une action de l'utilisateur sur une interface utilisateur (112, 212, 222) du système de prothèse auditive ou un signal de message d'état indiquant un changement dans une condition du système de prothèse auditive (100).

3. Système selon l'une des revendications précédentes, le signal audio d'interaction d'utilisateur étant sélectionné de manière à être perceptible par le patient comme un son standard associé au message de rétroaction respectif ou au message d'état respectif.

4. Système selon l'une des revendications 2 et 3, le signal de message d'état indiquant une connexion ou une déconnexion sans fil de l'unité de traitement (108) avec un dispositif externe (222) ou un état de batterie faible.

5. Système selon l'une des revendications 2 et 4, le signal de message de rétroaction indiquant une action d'utilisateur entraînant un changement de programme auditif, une augmentation ou une réduction de volume ou un verrouillage/déverrouillage d'implant.

6. Système selon l'une des revendications 2 à 5, le signal de message de rétroaction étant une réponse à une action d'utilisateur sur une commande d'opérateur (212, 222) .

7. Système selon l'une des revendications précédentes, l'unité de traitement (108) étant configurée pour appliquer la stimulation auditive perceptible correspondant au signal audio d'interaction d'utilisateur à un niveau situé dans la plage de confort.

8. Système selon l'une des revendications précédentes, le signal audio d'interaction d'utilisateur comprenant des tonalités sinusoïdales et/ou des balayages de fréquence.

9. Système selon l'une des revendications précédentes, l'enregistrement de la réponse neuronale au signal audio d'interaction d'utilisateur comprenant des mesures de seuil d'imagerie de réponse neuronale (NRI), des mesures de seuil d'électrocochléographie (ECochG), des mesures de réponse corticale et des mesures d'impédance d'électrode.

10. Système selon l'une des revendications précédentes, l'unité de traitement (108) étant configurée pour appliquer ladite stimulation auditive perceptible au patient en fonction du signal audio d'interaction d'utilisateur sous forme de stimulation électrique par l'intermédiaire du réseau d'électrodes (106).

11. Système selon l'une des revendications précédentes, le système de prothèse auditive (100) étant un système de stimulation électroacoustique, EAS, comprenant un transducteur de sortie électroacoustique (110), et l'unité de traitement étant configurée pour appliquer ladite stimulation auditive perceptible au patient en fonction du signal audio d' interaction d'utilisateur sous forme de stimulation acoustique par l'intermédiaire du transducteur de sortie électroacoustique.

12. Système selon la revendication 11, l'enregistrement de la réponse neuronale au signal audio d'interaction d'utilisateur comprenant la mesure de signaux ECochG.

13. Système selon l'une des revendications précédentes, l'unité de traitement (108) étant configurée pour ajuster automatiquement des paramètres d'adaptation du système de prothèse auditive (100) en fonction de la réponse neuronale enregistrée.

14. Système selon la revendication 13, l'unité de traitement (108) étant configurée pour ajuster automatiquement des niveaux de seuil de stimulation du système de prothèse auditive (100) en fonction de la réponse neuronale enregistrée, et le signal audio d'interaction d'utilisateur entraînant une stimulation auditive adaptée à la détermination du seuil respectif à partir de la réponse neuronale enregistrée.

15. Système selon la revendication 14, l'enregistrement de la réponse neuronale au signal audio d'interaction d'utilisateur comprenant des mesures de la réponse corticale pour l'ajustement de seuils de stimulation électrique.
